# EUROPEAN PATENT APPLICATION

(11) **EP 4 616 844 A1**
(43) Date of publication of application: **17.09.2025**
(21) Application number: 25162723.8
(22) Date of filing: 10.03.2025
(51) Int. Cl.: A61K 9/00, A61K 47/10, A61K 47/14, A61P 17/02, A61K 31/14, A61K 33/20

(54) **TOPICAL ANTIMICROBIAL LOTION FOR TREATING PYOTRAUMATIC DERMATITIS LESIONS**

(30) Priority: 09.03.2024 US 202418600654
(71) Applicant: Shear/Kershman Laboratories, Inc., Chesterfield, MO 63005 (US)
(72) Inventor: KERSHMAN, Alvin, St. Louis, MO 63146 (US); SHEAR, Jeff, Bonita Springs, FL 34135 (US); LINZE, Doreen, Labadie, MO 63055 (US)
(74) Representative: Dentons Patent Solutions Rechtsanwaltsgesellschaft mbH

(57) **Abstract**

An alcohol-free lotion for the topical application for treating *Pyotraumatic dermatitis* lesions is made by combining an aqueous phase comprising water, an antimicrobial active, and at least one humectant with an oil phase, wherein the at least one humectant is present in the aqueous phase in the range of from 5 to 30 wt.%., wherein the oil phase comprises an oil soluble preservative, at least one surfactant, and at least one oil, wherein the surfactant is present in the oil phase in the range of from about 20 to 60 wt. %, and wherein the aqueous phase to oil phase ratio is from about 12:1 to 1:2.

## Description

### CROSS-REFERENCE TO RELATED APPLICATIONS

This application is the first filing of this invention.

### STATEMENT REGARDING FEDERALLY SPONSORED RESEARCH

Not Applicable.

### APPENDIX

Not Applicable.

### Technical Field

The present invention relates to a topical antimicrobial lotion suitable for treating skin lesions. More specifically, this invention relates to a topical lotion to treat *pyotraumatic dermatitis* lesions on dogs.

### Background of the Invention

Canine *pyotraumatic dermatitis* or acute moist dermatitis, also known as "hot spots" are red, inflamed skin lesions that appear quickly, ooze, and may contain pus. Hot spots can be found anywhere on a dog's body, but the most common sites are the head, legs, and hips.

Hot spots are usually caused by self-trauma when a dog scratches an itch so vigorously that it creates an open wound. Many things can cause the initial itch in dogs including allergies.

When a dog licks the sore spot, it irritates superficial nerve endings in the skin which stimulates more itching followed by more licking, biting, and scratching. This lick-itch-lick cycle is the basis for the self-trauma that causes hot spots. Hot spots can dramatically increase in size in a very short period of time.

Hot spots have traditionally be treated with antibiotics. However, with the rise of biological antimicrobial resistance in small animal clinical practice, topical antimicrobial application is preferred for the management of superficial bacterial infections. Recent studies now support recommendations to use antimicrobials as the sole treatment of uncomplicated superficial skin infections. However, some antimicrobial actives, such as formaldehyde and metal compounds, such as stannous fluoride, zinc sulfate, and copper compounds have environmental disadvantages and are undesirable to treat *Pyotraumatic dermatitis* lesions .

The present invention is a topical lotion to treat *Pyotraumatic dermatitis* lesions. The lotion has antimicrobial properties but does not contain actives with undesirable environmental or biological side effects.

U.S. 8,586,102 discloses an antimicrobial lotion that uses stannous fluoride and zinc sulfate. U.S. 9,173,941 discloses a lotion to deter animal wound licking. U.S. 11,484,483 a hand sanitizer lotion.

None of the above patents disclose the claimed invention.

### SUMMARY OF THE INVENTION

The present invention is a homogeneous topical lotion wherein the lotion contains from about 1.0 to 10 wt. % surfactant, from about 1.0 to 20.0 wt. % oil, from about 4.0 to 30.0 wt. % humectant, from about 0.01 to 5.0 wt. % gelling agent, from about 30 to 80 wt. % water, and from about 1.0 to 3.0 oil soluble preservative, from about 0.1 to 10.0 wt. % antimicrobial agent. In a more preferred embodiment, the lotion also contains a bittering agent.

The present invention is made by combining an aqueous phase comprising water and at least one humectant with an oil phase to form a topical lotion with hydrophobic properties. The at least one humectant is present in the aqueous phase in the range of from about 5.0 to about 30.0 wt. %. In a preferred embodiment, the at least one humectant is present in the aqueous phase from about 7.0 to 27.0 wt. %. The oil phase comprises at least one surfactant and at least one oil. The surfactant is present in the oil phase in the range of from about 20 to 60 wt. %. In a preferred embodiment, the surfactant is present in the oil phase from about 20 to 50 wt. %. The aqueous phase is added to the oil phase in a weight ratio of about 12:1 to 1:2. The aqueous phase is added to the oil phase using low to medium shear mixing to provide the homogeneous hydrophobic topical lotion. The lotion is stable, hydrophobic, and has antiviral properties.

It should be understood that the detailed description and specific examples, while indicating the preferred embodiment of the invention, are intended for purposes of illustration only and are not intended to limit the scope of the invention.

### DETAILED DESCRIPTION OF THE PREFERRED EMBODIMENTS

The following description of the preferred embodiment(s) is merely exemplary in nature and is in no way intended to limit the invention, its application, or uses.

The oil phase is prepared from a hydrophobic solution or mixture containing optionally at least one oil and/or petroleum distillate and at least one surfactant. The surfactant is preferably a non-water soluble surfactant having an HLB number of less than about 6, and includes emulsifiers. A preferred surfactant is commercially sold as ATMOS^{®} 300K, and is a combination of mono- and di-glycerides made from edible food sources and propylene glycol with an HLB of 2.8. Another preferred surfactant is Defospum E100, a defoamer which is a combination of mono- and di-glycerides sold by Defotec. A third preferred surfactant is Lucrafoam E100, a defoamer which is a combination of mono-, di- and tri-glycerides sold by Levaco Chemicals.

It was determined that a surfactant that contained a combination of mono- and di-glycerides without propylene glycol, such as Lamchem^{®} PE-130K failed to create a stable lotion and was unsuitable for the present invention.

In a preferred embodiment, the oil phase contains a second surfactant that has an HLB number of about 6. The preferred surfactant is glyceryl monostearate and has an HLB of about 5.8. In a more preferred embodiment, the ratio of ATMOS^{®}300K surfactant to the glyceryl monostearate is from about 50:1 to about 1:1. In a preferred embodiment, the, ratio of ATMOS^{®}300K surfactant to the glyceryl monostearate is from about 40:1 to about 1:1. The total surfactant is present in the oil phase in the amount of about 20 to 60 wt. %, and the oil is present in the oil phase from about 80 to 40 wt. %.

A third surfactant is benzylalkonium chloride, a water soluble cationic surfactant, which also provides antimicrobial properties. This surfactant is dissolved in the aqueous phase. Cationic surfactants cannot be used with anionic surfactants in this composition as the combination is unstable and often precipitates. The combination of three surfactants, e.g., ATMOS^{®}300k, glyceryl monostearate and benzylaknomium chloride, is preferred.

Certain surfactants are not compatible with the present invention, and cause the lotion to fail, in that the continuous phase oil enveloping the aqueous phase either doesn't form or quickly breaks down. For example, the Tween surfactants, having a hydrophilic ethylene glycol head and hydrophobic alkyl tail, cause the lotion to fail. Tween 20 is a polysorbate monooleate nonionic surfactant that causes the lotion to break down into separate phases.

The oil suitable for the oil phase is typically liquid or semi-solid at room temperature, and is compatible with the topical applications. Such oils include essential oils, plant oils, such as vegetable oil, corn oil, canola oil, coconut oil, castor oil or olive oil, shea butter, and animal fats such as tallow and lard. The oils also include petroleum distillates, such as petrolatum and mineral oil. A preferred oil is mineral oil, such as Citation 70. Mixtures of oils are also contemplated in the present invention. The oil phase is present in the lotion in the range of from about 5 to 30 wt. %. In a preferred embodiment, the surfactant and the oil are present in the lotion in a weight ratio of about 1:4 to 1:1 surfactant to oil. Oils that act as emollients are also suitable for the present invention, and include vitamin E. In a preferred embodiment, the lotion contains from about 0.1 to 10.0 wt. % vitamin E.

Certain oils are not suitable for the present lotion. This includes castor oil, which has triglycerides having about 90% fatty acid chains from ricinoleats. Oleates and lioleates are other significant components of castor oil.

Preferably, the lotion of the present invention has an oil soluble preservative. Preservatives are antimicrobial ingredients added to product formulations to maintain the microbiological safety of the products by inhibiting the growth of and reducing the amount of microbial contaminants. The antimicrobial preservatives include essential oils with germicidial properties. Lexguard^{®}Natural MB preservative is a natural preservative made of high purity caprylic monoesters and undecylenic acid. VegeCide^{®} natural preservative is made from high purity sources of caprylic and undecylenic fatty acids. It contains glyceryl monocaprylate, and glyceryl monoundecylenate. Both are oil soluble and considered useful when added to the oil phase of a product. Other preservatives include potassium sorbate. Antioxidants, such as ascorbic acid and Vitamin E act as preservatives and may also be added to prevent oxidation and improve the shelf-life of the product.

Not all preservatives are compatible with the present invention. Alcohols such as methanol, ethanol, propanol, isopropanol, and butanol will cause the lotion to fail. Preservative alcohols such as benzyl alcohol and phenoxyethanol cause the lotion to separate into phases and fail. However, sugar alcohols, such as fructose and sorbitol, will not adversely affect the lotion.

The aqueous phase contains at least one humectant. Suitable humectants include, but are not limited to glycerin, lactic acid, polyols, propylene glycol, corn syrup, high fructose corn syrup (HFCS), including Cornsweet 55 (55 wt. % fructose, 24 wt. % water and 21 wt. % glucose) and Cornsweet 42 (42 wt. % fructose, 24. wt.% water and 34 wt. % glucose), and sorbitol. The at least one humectant is present in the aqueous phase from 2 to 86 wt. %. Preferably, the amount of humectant in the lotion is from about 5 to 50 wt. %. More preferably, the humectant is present from about 5 to 30 wt. %.

A preferred humectant is glycerin, which works to stabilize the lotion. More preferably the humectants in the lotion are from about 5 to 30 wt. % glycerol, and from about 1 to 15 wt. % sorbitol solution. A preferred sorbitol solution is non-crystalizing liquid sorbitol 70 wt. % in water.

Preferably, the aqueous phase also contains at least one antimicrobial as the active ingredient. Suitable antimicrobial agents include salts of chlorhexidine, such as chlorhexidene digluconate, chlorhexidene acetate, chlorhexidene isethionate, chlorhexidene hydrochloride. Other cationic antimicrobials may also be used, such as benzalkonium chloride (which also functions as a cationic surfactant), benzethonium chloride, polyhexamethylene biguanide, cetyl puridium chloride, methyl and benzothonium chloride. A preferred antimicrobial active is benzalkonium chloride, and a preferred concentration is from about 0.01 to about 0.3 wt. %.

Oxidative disinfectants share the property of acting as antimicrobial agents by oxidizing their proteins and cells. Such antimicrobial agents include hydrogen peroxide and hypochlorous acid provide an antimicrobial effect which is desirable. Preferably, the hydrogen peroxide is present in the lotion from about 0.150 wt. % to 3.0 wt. %. More preferably, the hydrogen peroxide is present from greater than about 0.125 wt. % to 2.0 wt. %. Hypochlorous acid, a source of hypochlorite ions and hydrogen ions, is present from about 0.010 to 0.100 wt. %.

A preferred combination is benzylalkonium chloride and either hydrogen peroxide or hypochlorous acid.

Various gelling agents can be employed including, for example and without limitation, starch and starch derivatives, cellulose derivatives, such as microcrystalline cellulose, sodium caboxymethyl cellulose, methylcellulose, ethyl cellulose, hydroxyethyl cellulose, hydroxypropyl cellulose, and hydroxypropyl methylcellulose, attapulgites, bentonites, dextrins, alginates, carrageenan, gum tragacanth, gum acacia, guar gum, xanthan gum, pectin, gelatin, kaolin, lecithin, the carbomers and carbopols, polyvinylpyrrolidone, polyethylene glycol, polyethylene oxide, polyvinyl alcohol, other polymeric materials, and mixtures thereof, etc. The pectin or pectic substances useful for this invention include not only purified or isolated pectates but also crude natural pectin sources, such as apple, citrus or sugar beet residues which have been subjected, when necessary, to esterification or de-esterification, e.g., by alkali or enzymes. Preferably, the pectins used in this invention are derived from citrus fruits such as lime, lemon, grapefruit, and orange. Preferred gelling agents are hyaluronic acid or its salt, carboxymethyl cellulose (CMC), guar gum, and a combination of guar gum and xanthan gum in the range of about 0.01 to 2.0 wt. %.

The claimed composition is typically prepared using a planetary or counter rotating type mixer having a rubber lined mixing bowl equipped with a wire whip stirring device. Preferably, the wire whip is rubber coated. The aqueous phase is blended at relatively low shear (30-600 rpm's) into the oil phase, continuously forming a total encapsulation of the aqueous solution droplets by the oil. This process is enhanced significantly by the oil wet-able properties of the rubber lining of the mixing bowl. Rubber coating of the wire whip device improves the rate of processing.

In an embodiment of the invention, the process of preparing the lotion is conducted as follows:
***Aqueous Phase:***
   1. Into a suitable container is measured the hyaluronic acid, sufficient water to make the desired concentration of hyaluronic acid mixture, and humectant (glycerin and/or sorbitol solution). The ingredients are mixed well.
***Oil Phase:***
   1. Accurately weigh all oil phase ingredients (oil(s) and surfactant(s)) into the kitchen aid bowl.
   2. Warm ingredients until homogeneously melted (10°F above the highest melt point ingredient). Reduce the heat.
***Mixing:***
   1. Keep the kitchen aid mixing on low speed and relatively low shear (30-600 rpm's) with the oil phase ingredients melted.
   2. Add the active to the oil phase and mix about 1 minute.
   3. Gradually, begin adding the Aqueous Phase ingredients to the bowl while continuing to mix at low speed while continuing to mix for 5 min after addition is complete.
   4. Stop the mixer and scrap down the bowl and mixing blade.
   5. Continuing mixing at speed 2 for an additional ten (10) minutes to produce the hydrophobic lotion.

The components of the hydrophobic lotion have the following preferred wt. % ranges:
***Oil Phase:***

| | |
|---|---|
| Surfactant: | 1.0 - 10.0 |
| Oil: | 1.0 - 20.0 |
| Oil soluble Preservative: | 0.1 - 3.0 |

***Aqueous Phase:***

| | |
|---|---|
| Humectant: | 4.0 - 30.0 |
| Gelling agent: | 0.01 - 5.0 |
| Water: | 30.0 - 80.0 |
| Active -Antimicrobial Agent: | 0.01 - 10.0 |

***In a preferred embodiment, the wt.% ranges for specific components are:***

| | |
|---|---|
| ATMOS^{®}300 | 1.0 - 25.0 |
| Glyceryl Stearate | 0.1 - 5.0 |
| Vitamin E | 0.1 - 10.0 |
| Citation 70 mineral oil | 2.0 - 26.0 |
| Oil soluble preservative | 0.1 - 10.0 |
| Glycerin | 4.0 - 30.0 |
| Sorbitol | 4.0 - 30.0 |
| Hyaluronic Acid | 0.1 - 2.0 |
| Water | 5.0 - 80.0 |
| Benzalkonium Chloride | 0.01 - 10.0 |
| Ascorbic Acid | 0 - 3.0 |
| Hydrogen Peroxide | 0 - 1.0 |
| Hypochlorous acid | 0 - 0.10 |

In a second embodiment of the process of this invention, the lotion is prepared as a first step and a second step. The first step produces a seed batch for further processing. The initial seed batch is produced by adding a small volume of oil phase to the lined mixing chamber or bowl at a sufficient depth that the wire whip or mixing device touches the oil while rotating. The wire whip is then engaged at rate of about 30 to100 rpm's. The aqueous phase is added at a rate approximately equivalent to the initial volume of the oil solution per minute. That is, if the initial volume of the oil phase is 20 mL, then the aqueous phase is added at a rate of about 20 mL per minute while being mixed in at 30 to100 rpm's. Once, the desired weight ratio of aqueous phase to oil phase is reached (about 12:1 to 1:2), this initial process step is concluded.

The second step begins with the seed batch of the first step, at the desired final weight ratio of aqueous phase to oil phase. The volume of seed material needed for the second step is to about 5-20 volume % of the final mixing chamber volume. The mixing whip or equivalent stirring and folding device are engaged at a speed of about 50 to 600 rpm's. The oil and water phases are added separately and simultaneously to the starter batch at a ratio equal to that contained in the seed batch. The rate of adding the two separate solutions is about 1 to 5% of the mixing chamber capacity per minute. As the mixing bowl or chamber fills, excess liquid may be removed continuously without halting the process. Alternatively, the process can be halted for partial or entire contents removal. Once the process is halted and a portion of the contents removed, the retained material can be held for an extended period of time. Because coating of and encapsulation of the aqueous phase is almost immediate, and materials are mixed at final required ratio in step 2, all product produced at any time during step 2 is ready to use.

### Stability Testing

The lotion of the present invention is evaluated for stability by at least two (2) visual tests. In the first test, a small amount of lotion is spread in a thin layer on a microscope slide and observed periodically for about 24 hours. The layer is examined under a microscope at 10 to 40 times enlargement. It is examined for smoothness and glossiness, which indicates a successful formulation. If it is observed that the surface has cracking, pooling or pitting, then failure is determined. Also, signs of separating and the formation of two phases also indicates formulation failure.

For a second test, a cup is filled with deionized water and a portion of lotion is dropped into the water and submerges. The lotion is observed periodically for about 24 hour. A successful formulation for lotion expands (grows in size) due to absorbing water, intact, while submerged in the water. A failure includes lotion dissolving completely in the water, or floating on top of the water, or breaking into pieces which break when moved with spoonula.

Another test of the lotion is hand feel. The lotion should be non-greasy, non-drippy, yet smooth and silky feeling on the skin.

A sample was prepared in Control 1, following the procedure below.

### Procedure to prepare the Lotion

**A. Preparing the Aqueous Phase (80.6 wt.% of the final lotion):**
1. Mix sufficient water and hyaluronic acid to make a 1.0 wt. % mixture. Add the glycerin, sorbitol, and optional additives. Mix well.
**B. Preparing the active:**
1. In a separate container, mix the active (benzalkonium chloride or hypochlorous acid) and the remainder of the water.
**C. Preparing Oil Phase (19.4 wt.% of final lotion):**
1. Mix the citation 70 mineral oil, surfactants, and preservative. Optionally add the vitamin E.
2. Heat the mixture with stirring to 155°F to form a homogeneous mixture. Reduce the heat to 130°F.
**D. Forming the lotion:**
1. Using a 5 quart lab Hobart type planetary lab/kitchen mixer with a rubber or plastic lined wire whip and rubber or plastic lined bowl add the oil phase and stir on #1 (low) setting.
2. Slowly, with continuous stirring, add the water/hyaluronic acid/ glycerin/sorbitol mixture alternating with the active (benzalkonium chloride and/or hypochlorous acid)/water mixture until all the aqueous phase is added.
3. Continue mixing for 5 minutes, turn off the mixer, and scrape the sides of the bowl with a spatula to ensure thorough mixing.
4. Continue to mix for 10 minutes more on setting #2, making sure to scrape the sides of the bowl occasionally. After 10 minutes of mixing, the lotion is prepared.

| **Control 1 - with HOCl, BAC and Lecithin** | | |
|---|---|---|
| **Ingredient** | **Weight** | **%** |
| **Oil Phase** | | |
| Yelkin^{®} Gold Lecithin surfactant | **18.00** | 9.00% |
| Citation 70 mineral oil | **12.734** | 6.367% |
| LEXGARD^{®} NATURAL MB preservative | **3.00** | 1.50% |
| Vitamin E 1300IU (Natural Source) | **1.00** | 0.50% |
| Glyceryl Stearate surfactant | **4.00** | 2.00% |

| **Aqueous Phase** | | |
|---|---|---|
| Benzalkonium Chloride 95% (BAC) active | **0.266** | 0.133% |
| Hyaluronic Acid gelling agent | **0.98** | 0.49% |
| Shield^{™} Sanitizer - 0.05% hypochlorous acid (HOCl) active | **20.00** | 0.5%HOCl |
| | | 9.5% water |
| Sorbitol Solution 70% USP humectant | **50.00** | 17.5% sorbitol |
| | | 7.5% water |
| Glycerin, USP 99.7% humectant | **18.00** | 9.00% |
| purified water (Distilled) | **72.00** | 36.00% |
| Denatonium Benzoate bittering agent | **0.02** | 0.01% |
| | | |
| | | |
| ***Totals*** | **200** | **100.00000000%** |

The lotion of Control 1 was unstable after about a week.

| **Control 2 - HOCl, BAC and Lamchem PE-130** | | |
|---|---|---|
| **Ingredient** | **Weight** | **%** |
| **Oil Phase** | | |
| Lamchem PE-130 K surfactant | **15.00** | 6.00% |
| citation 70 mineral oil | **23.418** | 9.367% |
| LEXGARD^{®} NATURAL MB | **3.75** | 1.50% |
| Vitamin E 1300IU (Natural Source) | **1.25** | 0.50% |
| Glyceryl Stearate (GMS) | **5.00** | 2.00% |

| **Aqueous Phase** | | |
|---|---|---|
| Benzalkonium Chloride 95% | **0.333** | 0.133% |
| Hyaluronic Acid Pure Powder | **1.23** | 0.49% |
| Shield Sanitizer 0.05% HOCl | **25.00** | 0.5% HOCl, 9.50% water |
| Sorbitol Solution 70% USP | **56.25** | 15.75% sorbitol |
| | | 6.75% water |
| Glycerin, USP 99.7% Excipient/Food use | **56.25** | 22.50% |
| purified water (Distilled) | **62.50** | 25.00% |
| Denatonium Benzoate Powder | **0.03** | 0.01% |
| | | |
| | | |
| ***Totals*** | **250** | **100.00000000%** |

Control 2 started leaking in about a month.

| **Example 3** - **Atmos^{®} 300K Surfactant** | | |
|---|---|---|
| **Ingredient** | **Weight** | **%** |
| **Oil Phase** | | |
| Atmos 300K surfactant | **15.00** | 5.00% |
| Citation 70 mineral oil | **31.101** | 10.367% |
| LEXGARD^{®} NATURAL MB | **4.50** | 1.50% |
| Vitamin E 1300IU (Natural Source) | **1.50** | 0.50% |
| Glyceryl Stearate (GMS) | **6.00** | 2.00% |

| **Aqueous Phase** | | |
|---|---|---|
| Benzalkonium Chloride 95% | **0.399** | 0.133% |
| Hyaluronic Acid Pure Powder | **1.47** | 0.49% |
| Shield Sanitizer 0.05% HOCl | **30.00** | 0.5% HOCl |
| | | 9.50% water |
| Sorbitol Solution 70% USP | **67.50** | 22.50% |
| Glycerin, USP 99.7% Excipient/Food use | **67.50** | 15.75% sorbitol |
| | | 6.75% water |
| purified water (Distilled) | **75.00** | 25.00% |
| Denatonium Benzoate Powder | **0.03** | 0.01% |
| | | |
| | | |
| ***Totals*** | **300** | **100.00000000%** |

Example 3 was stable.

| **Example 4 - HOCL, Atmos 300K** | | |
|---|---|---|
| **Ingredient** | **Weight** | **%** |
| **Oil Phase** | | |
| Atmos 300K | **15.00** | 5.00% |
| citation 70 | **31.500** | 10.500% |
| LEXGARD^{®} NATURAL MB | **4.50** | 1.50% |
| Vitamin E 1300IU (Natural Source) | **1.50** | 0.50% |
| Glyceryl Stearate (GMS) | **6.00** | 2.00% |

| **Aqueous Phase** | | |
|---|---|---|
| Hyaluronic Acid Pure Powder | **1.47** | 0.49% |
| Shield Sanitizer 0.05% HOCl | **30.00** | 0.5% HOCl |
| | | 9.50% water |
| Sorbitol Solution 70% USP | **67.50** | 15.75% sorbitol |
| | | 6.75% water |
| Glycerin, USP 99.7% Excipient/Food use | **67.50** | 22.50% |
| purified water (Distilled) | **75.00** | 25.00% |
| Denatonium Benzoate Powder | **0.03** | 0.01% |
| | | |
| | | |
| ***Totals*** | **300** | **100.00000000%** |

Example 4 was stable.

| **Example 5** | | |
|---|---|---|
| **Ingredient** | **Weight** | **%** |
| **Oil Phase** | | |
| Atmos 300K | **15.00** | 5.00% |
| citation 70 | **31.101** | 10.367% |
| LEXGARD^{®} NATURAL MB | **4.50** | 1.50% |
| Vitamin E 1300IU (Natural Source) | **1.50** | 0.50% |
| Glyceryl Stearate (GMS) | **6.00** | 2.00% |
| Benzalkonium Chloride 95% | **0.399** | 0.133% |

| **Aqueous Phase** | | |
|---|---|---|
| Hyaluronic Acid Pure Powder | **1.47** | 0.49% |
| Sorbitol Solution 70% USP | **82.50** | 19.25% sorbitol, 8.25% water |
| Glycerin, USP 99.7% Excipient/Food use | **82.50** | 27.50% |
| purified water (Distilled) | **75.00** | 25.00% |
| Denatonium Benzoate Powder | **0.03** | 0.01% |
| | | |
| | | |
| ***Totals*** | **300** | **100.00000000%** |

Example 5 was stable.

| **Example 6 - BAC, Atmos 300K** | | |
|---|---|---|
| **Ingredient** | **Weight** | **%** |
| **Oil Phase** | | |
| Atmos 300K | **15.00** | 5.00% |
| citation 70 | **31.101** | 10.367% |
| LEXGARD^{®} NATURAL MB | **4.50** | 1.50% |
| Vitamin E 1300IU (Natural Source) | **1.50** | 0.50% |
| Glyceryl Stearate (GMS) | **6.00** | 2.00% |

| **Aqueous Phase** | | |
|---|---|---|
| Benzalkonium Chloride (BAC) 95% | **0.399** | 0.133% |
| Hyaluronic Acid Pure Powder | **1.47** | 0.49% |
| Sorbitol Solution 70% USP | **45.00** | 10.5% sorbitol |
| | | 4.5% water |
| Glycerin, USP 99.7% Excipient/Food use | **45.00** | 15.00% |
| purified water (Distilled) | **150.00** | 50.00% |
| Denatonium Benzoate Powder | **0.03** | 0.01% |
| | | |
| | | |
| ***Totals*** | **300** | **100.00000000%** |

Example 6 was stable.

| **Example 7 - BAC, Atmos 300K** | | |
|---|---|---|
| **Ingredient** | **Weight** | **%** |
| **Oil Phase** | | |
| Atmos 300K | **15.00** | 5.00% |
| citation 70 | **31.101** | 10.367% |
| LEXGARD^{®} NATURAL MB | **4.50** | 1.50% |
| Vitamin E 1300IU (Natural Source) | **1.50** | 0.50% |
| Glyceryl Stearate (GMS) | **6.00** | 2.00% |

| **Aqueous Phase** | | |
|---|---|---|
| Benzalkonium Chloride 95% | **0.399** | 0.133% |
| Hyaluronic Acid Pure Powder | **0.90** | 0.30% |
| Sorbitol Solution 70% USP | **30.00** | 7.50% sorbitol |
| | | 2.5% water |
| Glycerin, USP 99.7% Excipient/Food use | **30.00** | 10.00% |
| purified water (Distilled) | **180.57** | 60.19% |
| Denatonium Benzoate Powder | **0.03** | 0.01% |
| | | |
| | | |
| ***Totals*** | **300** | **100.00000000%** |

Example 7 was stable.

| **Example 8** | | |
|---|---|---|
| **Ingredient** | **Weight** | **%** |
| **Oil Phase** | | |
| Atmos 300K | **15.00** | 5.00% |
| citation 70 | **31.101** | 10.367% |
| LEXGARD^{®} NATURAL MB | **4.50** | 1.50% |
| Vitamin E 1300IU (Natural Source) | **1.50** | 0.50% |
| Glyceryl Stearate (GMS) | **6.00** | 2.00% |

| **Aqueous Phase** | | |
|---|---|---|
| Benzalkonium Chloride 95% | **0.399** | 0.133% |
| Hyaluronic Acid Pure Powder | **0.60** | 0.20% |
| Sorbitol Solution 70% USP | **15.00** | 3.50% sorbitol |
| | | 1.5% water |
| Glycerin, USP 99.7% Excipient/Food use | **15.00** | 5.00% |
| purified water (Distilled) | **210.87** | 70.29% |
| Denatonium Benzoate Powder | **0.03** | 0.01% |
| | | |
| | | |
| ***Totals*** | **300** | **100.00000000%** |

### Example 8 was stable

Determining the Efficacy of Example 7 against *Staphylococcus pseudointermedius.*

*Staphylococcus pseudointermedius* was tested against the lotion of Example 7 at a concentration of 10 g lotion per 3.3 mL of bacterial suspension (concentration 10⁶) along with a diluent blank and positive and negative controls to determine antimicrobial activity. The results are in Table 1, below.

**Table 1 - Results of Test**

| | **Pretiter CVU** | **Postive Control** | **Sample CFU/mL 0 hour** | **Sample CFU/mL 6 hour** | **Sample CFU/mL 12 hour** | **Sample CFU/mL 24 hour** |
|---|---|---|---|---|---|---|
| **Results** | 1.6 X 10⁸ | TNTC | 2.3 X 10⁶ | 6.0 X 10⁴ | 2.0 X 10² | 2.0 X 10² |

Regarding the results in Table 1, the log reduction from 0 hour to 6 hour is 1.58, and the percent reduction is 97.39%. The log reduction from 6 to 12 hours is 2.48 and the percent reduction is 99.67%. The log reduction from 0 to 24 hours is 4.06 and the percent reduction is 99.99%.

The efficacy of the claimed lotion in reducing *Staphylococcus pseudointermedius* is clearly shown with the results in Table 1.

These embodiments were chosen and described to best explain the principles of the invention and its practical application to persons who are skilled in the art. As various modifications could be made to the exemplary embodiments, as described above with reference to the corresponding illustrations, without departing from the scope of the invention, it is intended that all matter contained in the foregoing description and shown in the accompanying drawings shall be interpreted as illustrative rather than limiting. Thus, the breadth and scope of the present invention should not be limited by any of the above-described exemplary embodiments, but should be defined only in accordance with the following claims appended hereto and their equivalents.

### CLAUSES

The present invention may also be described by the following clauses:
1. A lotion for topical application for treating *Pyotraumatic dermatitis* lesions made by the process of combining:
A) an aqueous phase comprising water, at least one humectant, and water;
   wherein the at least one humectant is present in the aqueous phase in the range of from about 5 to 30 wt. %;
B) an oil phase comprising at least one surfactant, at least one oil, and at least one oil soluble preservative;
   wherein the at least one surfactant is present in the oil phase in the range of from about 20 to 60 wt.%;
   wherein the at least one surfactant comprises mono- and di-glycerides made from edible food sources and propylene glycol with an HLB of less than about 6; and
C) at least one antimicrobial active;
   wherein the aqueous phase is added to the oil phase in a weight ratio of about 12:1 to 1:2,
   and the aqueous phase is added to oil phase using low to medium shear mixing to provide the lotion;
   wherein the lotion does not contain polysorbate monooleate nonionic surfactants;
   wherein the lotion is hydrophobic;
   wherein the lotion is alcohol free, antibiotic free, metal compound free, and formaldehyde free; and
   wherein the lotion has antimicrobial properties.
2. The lotion of clause 1, wherein the aqueous phase further comprises a gelling agent.
3. The lotion of clause 1 or clause 2, wherein the at least one surfactant further comprises glyceryl monostearate;
wherein the ratio of mono- and di-glycerides made from edible food sources and propylene glycol with an HLB of less than 6 to the glyceryl monostearate is from about 5:1 to about 1:1;
wherein the total surfactant is present in the oil phase in the amount of about 20 to 60 wt. %; and
wherein the oil is present in the oil phase from about 80 to 40 wt. %.
4. The lotion of any one of clauses 1 to 3, wherein the at least one antimicrobial active is selected from the group consisting of benzylalkonium chloride and hypochlorous acid.
5. The lotion of any one of clauses 1 to 4, wherein the antimicrobial active is benzylalkonium chloride.
6. The lotion of any one of clauses 1 to 5, wherein the oil soluble preservative comprises caprylic acids and esters, and undecylenic fatty acids and esters.
7. The lotion of any one of clauses 1 to 6, wherein the at least one antimicrobial active comprises a cationic surfactant, and wherein the lotion does not contain an anionic surfactant.
8. A lotion for topical application for treating *Pyotraumatic dermatitis* lesions made by the process of:
adding to a container a gelling agent to water, shaking, and cooling to form a viscous aqueous solution;
after about 12 h, mixing at least one humectant with the viscous aqueous solution to form an aqueous mixture;
wherein the at least one humectant is present in the aqueous mixture in the range of from about 5 to 30 wt. %;
adding to a mixing container at least one oil, at least one surfactant, and an oil soluble preservative to form an oil mixture;
wherein the at least one surfactant is present in the oil mixture in the range of from about 20 to 60 wt.%;
warming the oil mixture until melted forming a warm oil mixture;
mixing the warm oil mixture using low shear mixing;
adding to the warm oil mixture an antimicrobial active and mixing for about one minute;
slowly adding the aqueous mixture to the mixing container while continuing to mix with low to medium shear mixing; and
continue mixing until both the warm oil mixture and the aqueous mixture combine to form one phase;
wherein the one phase is an alcohol-free lotion having sanitizing properties;
wherein the at least one surfactant comprises glyceryl monostearate, and mono- and di-glycerides made from edible food sources and propylene glycol with an HLB of less than 6;
wherein the ratio of mono- and di-glycerides made from edible food sources and propylene glycol with an HLB of less than 6 to the glyceryl monostearate is from about 5:1 to about 1:1;
wherein the total surfactant is present in the oil phase in the amount of about 20 to 60 wt. %;
wherein the oil is present in the oil phase from about 80 to 40 wt. %;
wherein the lotion does not contain polysorbate monooleate nonionic surfactants; and
wherein the lotion is alcohol free, antibiotic free, metal compound free, and formaldehyde free.
9. The lotion of clause 8, wherein the gelling agent comprises hyaluronic acid.
10. The lotion of clause 8 or 9, wherein the at least one surfactant is selected from the group consisting of monoglycerides, diglycerides, triglycerides, propylene glycol, and combinations thereof.
11. The lotion of any one of clauses 8 to 10, wherein the at least one antimicrobial active is selected from the group consisting of benzylalkonium chloride, and hypochlorous acid and combinations thereof; and wherein the lotion does not contain an anionic surfactant.
12. The lotion of any one of clauses 8 to 11, wherein the oil phase contains vitamin E.
13. The lotion of any one of clauses 8 to 12, wherein the oil soluble preservative comprises caprylic acids and esters, and undecylenic fatty acids and esters.
14. The lotion of any one of clauses 8 to 13, wherein oil soluble preservative comprises caprylic monoesters and undecylenic acid.
15. A lotion for topical application for treating *Pyotraumatic dermatitis* lesions comprising:

| **Component** | **Wt. %** |
|---|---|
| Surfactant: | 1.0 - 10.0 |
| Oil: | 1.0 - 20.0 |
| Oil soluble Preservative: | 1.0 - 3.0 |
| Humectant: | 4.0 - 30.0 |
| Gelling agent: | 0.01 - 5.0 |
| Water: | 30.0 - 80.0 |
| Antimicrobial Agent: | 0.01 - 10.0 |

wherein the at least one surfactant comprises glyceryl monostearate and mono- and di-glycerides made from edible food sources and propylene glycol with an HLB of less than 6;
wherein the ratio of mono- and di-glycerides made from edible food sources and propylene glycol with an HLB of less than 6 to the glyceryl monostearate is from about 5:1 to about 1:1;
wherein the total surfactant is present in the oil phase in the amount of about 20 to 60 wt. %;
wherein the oil is present in the oil phase from about 80 to 40 wt. %;
wherein the lotion does not contain polysorbate monooleate nonionic surfactants;
wherein the lotion is homogeneous and hydrophobic;
wherein the lotion is alcohol free, antibiotic free, metal compound free, and formaldehyde free; and
wherein the lotion has germicidal properties.
16. The lotion of clause 15, wherein the at least one antimicrobial active comprises a cationic surfactant, and wherein the lotion does not contain an anionic surfactant.
17. The method of clause 15 or 16, wherein the at least one antimicrobial active is selected from the group consisting of benzylalkonium chloride and hypochlorous acid and combinations thereof.
18. The lotion of any one of clauses 15 to 17, wherein the at least one humectant is selected from the group consisting of glycerine, lactic acid, polyols, propylene glycol, high fructose corn syrup, and sorbitol and combinations thereof.
19. The lotion of any one of clauses 15 to 18, wherein the gelling agent is selected from the group consisting of hyaluronic acid, the salt of hyaluronic acid, carboxymethyl cellulose, guar gum, and a combination of guar gum and xanthan gum.
20. The lotion of any one of clauses 15 to 19, wherein the oil soluble preservative comprises caprylic monoesters and undecylenic acids.

## Claims

1. A lotion for topical application for treating *Pyotraumatic dermatitis* lesions made by the process of combining:
A) an aqueous phase comprising water, at least one humectant, and water;
wherein the at least one humectant is present in the aqueous phase in the range of from about 5 to 30 wt. %;
B) an oil phase comprising at least one surfactant, at least one oil, and at least one oil soluble preservative;
wherein the at least one surfactant is present in the oil phase in the range of from about 20 to 60 wt.%;
wherein the at least one surfactant comprises mono- and di-glycerides made from edible food sources and propylene glycol with an HLB of less than about 6; and
C) at least one antimicrobial active;
wherein the aqueous phase is added to the oil phase in a weight ratio of about 12:1 to 1:2,
and the aqueous phase is added to oil phase using low to medium shear mixing to provide the lotion;
wherein the lotion does not contain polysorbate monooleate nonionic surfactants;
wherein the lotion is hydrophobic;
wherein the lotion is alcohol free, antibiotic free, metal compound free, and formaldehyde free; and
wherein the lotion has antimicrobial properties.

2. The lotion of claim 1, wherein the aqueous phase further comprises a gelling agent.

3. The lotion of claim 1 or 2, wherein the at least one surfactant further comprises glyceryl monostearate;
wherein the ratio of mono- and di-glycerides made from edible food sources and propylene glycol with an HLB of less than 6 to the glyceryl monostearate is from about 5:1 to about 1:1;
wherein the total surfactant is present in the oil phase in the amount of about 20 to 60 wt. %; and
wherein the oil is present in the oil phase from about 80 to 40 wt. %.

4. The lotion of any one of claims 1 to 3, wherein the at least one antimicrobial active is selected from the group consisting of benzylalkonium chloride and hypochlorous acid, wherein optionally the antimicrobial active is benzylalkonium chloride.

5. The lotion of any one of claims 1 to 4, wherein the oil soluble preservative comprises caprylic acids and esters, and undecylenic fatty acids and esters.

6. The lotion of any one of claims 1 to 5, wherein the at least one antimicrobial active comprises a cationic surfactant, and wherein the lotion does not contain an anionic surfactant.

7. A lotion for topical application for treating *Pyotraumatic dermatitis* lesions made by the process of:
adding to a container a gelling agent to water, shaking, and cooling to form a viscous aqueous solution;
after about 12 h, mixing at least one humectant with the viscous aqueous solution to form an aqueous mixture;
wherein the at least one humectant is present in the aqueous mixture in the range of from about 5 to 30 wt. %;
adding to a mixing container at least one oil, at least one surfactant, and an oil soluble preservative to form an oil mixture;
wherein the at least one surfactant is present in the oil mixture in the range of from about 20 to 60 wt.%;
warming the oil mixture until melted forming a warm oil mixture;
mixing the warm oil mixture using low shear mixing;
adding to the warm oil mixture an antimicrobial active and mixing for about one minute;
slowly adding the aqueous mixture to the mixing container while continuing to mix with low to medium shear mixing; and
continue mixing until both the warm oil mixture and the aqueous mixture combine to form one phase;
wherein the one phase is an alcohol-free lotion having sanitizing properties;
wherein the at least one surfactant comprises glyceryl monostearate, and mono- and di-glycerides made from edible food sources and propylene glycol with an HLB of less than 6;
wherein the ratio of mono- and di-glycerides made from edible food sources and propylene glycol with an HLB of less than 6 to the glyceryl monostearate is from about 5:1 to about 1:1;
wherein the total surfactant is present in the oil phase in the amount of about 20 to 60 wt. %;
wherein the oil is present in the oil phase from about 80 to 40 wt. %;
wherein the lotion does not contain polysorbate monooleate nonionic surfactants; and
wherein the lotion is alcohol free, antibiotic free, metal compound free, and formaldehyde free.

8. The lotion of claim 7, wherein the gelling agent comprises hyaluronic acid.

9. The lotion of claim 7 or 8, wherein the at least one surfactant is selected from the group consisting of monoglycerides, diglycerides, triglycerides, propylene glycol, and combinations thereof.

10. The lotion of any one of claims 7 to 9, wherein the at least one antimicrobial active is selected from the group consisting of benzylalkonium chloride, and hypochlorous acid and combinations thereof; and wherein the lotion does not contain an anionic surfactant.

11. The lotion of any one of claims 7 to 10, wherein the oil phase contains vitamin E.

12. The lotion of any one of claims 7 to 11, wherein the oil soluble preservative comprises caprylic acids and esters, and undecylenic fatty acids and esters, and/or wherein oil soluble preservative comprises caprylic monoesters and undecylenic acid.

13. A lotion for topical application for treating *Pyotraumatic dermatitis* lesions comprising:
| **Component** | **Wt. %** |
|---|---|
| Surfactant: | 1.0 - 10.0 |
| Oil: | 1.0 - 20.0 |
| Oil soluble Preservative: | 1.0 - 3.0 |
| Humectant: | 4.0 - 30.0 |
| Gelling agent: | 0.01 - 5.0 |
| Water: | 30.0 - 80.0 |
| Antimicrobial Agent: | 0.01 - 10.0 |
wherein the at least one surfactant comprises glyceryl monostearate and mono- and di-glycerides made from edible food sources and propylene glycol with an HLB of less than 6;
wherein the ratio of mono- and di-glycerides made from edible food sources and propylene glycol with an HLB of less than 6 to the glyceryl monostearate is from about 5:1 to about 1:1;
wherein the total surfactant is present in the oil phase in the amount of about 20 to 60 wt. %;
wherein the oil is present in the oil phase from about 80 to 40 wt. %;
wherein the lotion does not contain polysorbate monooleate nonionic surfactants;
wherein the lotion is homogeneous and hydrophobic;
wherein the lotion is alcohol free, antibiotic free, metal compound free, and formaldehyde free; and
wherein the lotion has germicidal properties.

14. The lotion of claim 13, wherein the at least one antimicrobial active comprises a cationic surfactant, and wherein the lotion does not contain an anionic surfactant, and/or wherein the at least one antimicrobial active is selected from the group consisting of benzylalkonium chloride and hypochlorous acid and combinations thereof.

15. The lotion of claim 13 or 14, wherein the at least one humectant is selected from the group consisting of glycerine, lactic acid, polyols, propylene glycol, high fructose corn syrup, and sorbitol and combinations thereof, and/or wherein the gelling agent is selected from the group consisting of hyaluronic acid, the salt of hyaluronic acid, carboxymethyl cellulose, guar gum, and a combination of guar gum and xanthan gum, and/or wherein the oil soluble preservative comprises caprylic monoesters and undecylenic acids.
